Europäisches Patentamt

European Patent Office

Office européen des brevets

(11) Veröffentlichungsnummer: **0 499 710 A2**

# EUROPÄISCHE PATENTANMELDUNG

(21) Anmeldenummer: **91121304.9**

(51) Int. Cl.⁵: **A61F 13/06**

(22) Anmeldetag: **12.12.91**

(30) Priorität: **18.02.91 DE 4104930**

(43) Veröffentlichungstag der Anmeldung:
**26.08.92 Patentblatt 92/35**

(84) Benannte Vertragsstaaten:
**BE DE ES FR IT NL**

(71) Anmelder: **Beiersdorf Aktiengesellschaft**
**Unnastrasse 48**
**W-2000 Hamburg 20(DE)**

(72) Erfinder: **Brandt, Dieter**

**Wodanstrasse 5**
**W-4000 Düsseldorf(DE)**
Erfinder: **Szlema, Ingeborg M.A.**
**Kerkener Strasse 43**
**W-4152 Kempen(DE)**
Erfinder: **Wetz, Hans Henning, Dr. Med.**
**Ueriker Halde 8**
**CH-8713 Uerikon(CH)**

(74) Vertreter: **Richter, Werdermann & Gerbaulet**
**Neuer Wall 10**
**W-2000 Hamburg 36(DE)**

(54) **Sprunggelenk-Bandage.**

(57) Die Sprunggelenk-Bandage ermöglicht neben einer intermittierenden Kompression auf die Gelenkweichteile um den medialen und lateralen Malleolus eine tiefe Querfriktion auf den Ansatz der Achillessehne; sie besteht aus einem anatomisch geformten Schlauchkörper (11), der mit einem eine Laufsohle (16a) bildenden unteren Abschnitt (16) versehen ist und der im angelegten Zustand den halben Fuß umfaßt und bis zur Wade reicht, wobei im Bereich der Laufsohle (16a) eine halbe Ferse (18) eingestrickt ist und der zwei anatomisch asymmetrisch ausgebildete, über den paraachillären Weichteilen liegende und am medialen und lateralen Malleolus den Bereich über der Bisgaard'schen Kulisse abdeckende Druckpelotten (20) aufweist, von denen jede Druckpelotte als Formkörper (21) mit einer seitlichen, abgerundeten Aussparung (27) zur Aufnahme des Knöchelvorsprunges ausgebildet ist, der aus einem weichen oder weichelastischem Material besteht, wobei in dem Formkörper (21) ein stabförmiger Friktionskern (30) aus einem harten, inkompressiblen Material angeordnet und in dem Material des Formkörpers (21) in seiner Lage fixiert ist.

Fig.1

Die Erfindung betrifft eine Sprunggelenk-Bandage aus elastischem Bandagenstoff in Schlauchform.

Zum Stütz- und Bewegungsapparat zählend ist das komplizierte Sprunggelenk hohen Stoß- und Druckbelastungen ausgesetzt. Überbelastungen können in dieser Region schnell Weichteiltraumatisierungen hervorrufen. Oft führen Supinationstraumen infolge starker Überdehnung der Bänder bei feststehendem Fuß oder plötzliches Umknicken über den lateralen Fußrand zu Verletzungen im Sprunggelenk. Dabei treten Schäden unterschiedlicher Ausprägung mit der Gefahr der posttraumatischen Arthrose auf.

Das Sprunggelenk gilt in der Sportmedizin nach dem Kniegelenk als der am zweithäufigsten betroffene Verletzungsbereich. Frakturen und Bandläsionen des oberen Sprunggelenks nach Distorsionen sind die häufigsten Gelenkverletzungen des Menschen überhaupt.

Im Bereich des Fußes wird das obere und das untere Sprunggelenk unterschieden. Das enge Zusammenwirken beider sowie ein funktionierender Kapsel-Band-Apparat ist Voraussetzung für die Funktionsfähigkeit des Sprunggelenks. Das obere Sprunggelenk erlaubt als Scharniergelenk nur Streck- und Beugebewegungen. Schienbein (Tibia) und Wadenbein (Fibula) umfassen zangenförmig die Gelenkrolle des Sprungbeins (Trochlea tali) und bilden die Malleolengabel. Zusätzliche Stabilität geben das innere und äußere Kollateralband. Die Bänder sind in ihrer Verlaufstruktur gefächert, so daß - bei jeder Gelenkstellung - jeweils ein Teil angespannt ist.

Das untere Sprunggelenk hat einen erweiterten Bewegungsspielraum und läßt Drehbewegungen um eine von lateral hinten unten nach medial vorne oben gerichtete Schrägachse zu, so daß es die Pronation und Supination des Fußes ermöglicht. Da das Sprungbein auf dem Fersenbein aufliegt, wird zwischen dem vorderen und hinteren unteren Sprunggelenk unterschieden, welche funktionell eng zusammenwirken. Die Lücke zwischen Kahnbein und Fersenbein wird durch das Pfannenband geschlossen. Bei der Muskulatur des Fußes wird zwischen Muskeln mit primär statischen Aufgaben und Muskeln, die primär der Fortbewegung dienen, unterschieden. Die Muskulatur hat die wichtige Funktion, das Gelenk vor den einwirkenden Kräften zu schützen.

Der dreiköpfige Wadenmuskel (Musculus triceps surae), der sich aus dem Zwillingsmuskel (Musculus gastrocnemius) und dem Schollenmuskel (Musculus soleus) zusammensetzt, ist an der Plantarflexion beteiligt. Der lange Zehenbeuger (Musculus flexor digitorum longus) unterstützt außerdem im oberen Sprungelenk die Plantarflexion und ist im unteren Sprunggelnk an der Pronation beteiligt. Er stützt das Fuß-Längsgewölbe. Der hintere Schienbeinmuskel (Musculus tibias posterior) unterstützt im oberen Sprunggelenk die Plantarflexion und im unteren Sprunggelenk die Supination. Der Muskelansatz befindet sich am höchsten Punkt des Fußgewölbes, für dessen Längswölbung er verantwortlich ist. Der lange Großzehenbeuger (Musculus flexor hallucis) wirkt außerdem im oberen Sprunggelenk an der Plantarflexion mit. Er zieht sich unter dem Knochenvorsprung des Sprungbeins hindurch und arbeitet so dem Abknikken des Fersenbeins entgegen. Die Muskeln der Unterschenkelvorderseite sind im oberen Sprunggelenk an der Dorsalextension, im unteren Sprunggelenk an der Pronation beteiligt. Dazu gehören der lange Großzehenstrecker (Musculus extensor hallucis longus) und der lange Zehenstrecker (Musculus extensor digitorum longus). Der vordere Schienbeinmuskel (Musculus tibias anterior) unterstützt außerdem das Fußgewölbe. Der seitlich am Unterschenkel verlaufende lange Wadenbeinmuskel (Musculus peronaeus longus) und der kurze Wadenbeinmuskel (Musculus peronaeus brevis) haben im oberen Sprunggelenk plantarflektierende und im unteren Sprungelenk pronierende Wirkung. Die Achillessehne setzt sich aus den Sehnen des Musculus gastrocnemius und Musculus soleus zusammen. Als dickste und stärkste Sehne des Körpers reicht sie vom unteren Drittel der Wade bis zum Fersenbein. Ihr knöcherner Ansatz befindet sich an der distalen Hälfte des Tuber calcanei, wo sie breit nach medial und lateral ausfächert.

Durch die DE-A-38 40 714 ist eine Sprunggelenkorthese bekannt, die einen U-förmigen Stützbügel enthält, dessen Schenkel unterhalb des Fußes in einem Steg zusammenlaufen, über die Knöchel hinausreichen und in ihrem Endbereich durch ein Befestigungsband zusammengehalten sind. Der äußere Schenkel ist seitlich vor seinem Knöchel und der innere Schenkel in Gegenüberstellung zum äußeren Schenkel vor der Achillessehne hochgeführt. Die Schenkel sind in Richtung zum Steg bis zu einer Lage vor der Ferse geführt und verlaufen in Richtung zu ihren Enden aufwärts derart, daß sie seitlich neben den Schienbeinkanten etwa parallel zu diesen aufwärtsstreben. Im unteren Bereich der Schenkel ist ein Halteband, insbesondere Klettband, angebracht, das von dem einen Schenkel über den Rist schräg aufwärts zum anderen Schenkel an diesem festlegbar verläuft, oberhalb der Knöchel um die Achillessehne greift und auf dem Rist sich überkreuzend an dem anderen Schenkel in einem Halteteil endet. Mit einer derart ausgebildeten Sprunggelenkorthese soll das Umknicken vor allem in Richtung seitlichvorn, also in Richtung auf eine Spitzfuß-Stellung verhindert werden.

Die DE-U-G 85 03 139.9 betrifft eine Fußgelenk-Bandage, die im wesentlichen aus einem der Anatomie des Fußgelenks angepaßten Schlauchabschnitt aus elastischem Material besteht, wobei der der Achillessehne zuzuordnende Bereich mit einer Polstereinlage mit hoher Dämpfung und Rückstellkraft versehen ist. Die Polstereinlage selbst kann aus Kunststoff mit hoher Rückstellkraft und guter Formbeständigkeit, beispielsweise aus Polyform, bestehen. Mit einer derartigen Fußgelenk-Bandage soll die Achillessehne geschützt werden; gleichzeitig soll eine Fixierung der Schutzvorrichtung gewährleistet sein.

Die DE-A-38 38 582 beschreibt eine als gelenkbedeckende, elastische Knöchelsocke ausgebildete Gelenkbandage aus schlauchförmiger Strickware, wobei Form und Größe dem zu bandagierenden Sprunggelenk anatomisch angepaßt sind. In die Knöchelsocke ist ein U-förmiger Steigbügeleinsatz nach Art einer antagonistischen Schlinge integriert, der von einem um den Knöchel gelegten Anker spiralförmig umschlurgen wird, wobei der Anker als unelastisches Band mit Abstand seiner Windungen geführt ist und an dem äußeren Schenkel des Steigbügeleinsatzes beginnt und endet. Eine derart ausgebildete elastische Knöchelgelenkbandage soll unphysiclogische Dreh-, Scher- und Kippbewegungen abfangen und einen gesicherten sowie gerichteten funktionellen Bewegungsablauf im Sprunggelenk ermöglichen. Dabei soll die Bandage für das Sprunggelenk im wesentlichen drei Funktionen erfüllen, nämlich Schutz, Stabilisierung und Sportivität.

Zur Stützung erschlaffter Gewölbe und zu insuffizienter Muskel- und Bandführung des menschlichen Fußes sieht die DE-A-34 41 496 eine Vorrichtung vor, die aus einer fest am Fuß anliegenden Socke besteht, an deren Innen- oder Außenseite ein Gummizug so befestigt ist, daß er etwa quer unter der Fußsohle hindurch über den Spann zur Befestigungsseite zurückführbar und mit seinem freien Ende an der Socke festlegbar ist, wobei auf die Innen- oder Außenseite der Socke ein Streifen aus dehnfestem Material aufgenäht ist, der vom oberen Ende der Socke bis in die Nähe der Fußsohle reicht. Mit einer derartigen Vorrichtung soll eine Stützwirkung entsprechend den natürlichen Muskelzügen bewirkt werden, wobei je nach Spannung des Gummizugs eine stufenlose und progressive Entlastung der einzelnen Gewölbe möglich ist. Außerdem soll die Vorrichtung schuhunabhängig sein und somit einen dauerhaften Gebrauch auch nachts ermöglichen.

Die DE-A-39 24 428 beschreibt eine Stütze für den Sprunggelenkbereich, insbesondere zur seitlichen Abstützung des unteren Sprunggelenks mit einem als vorne offener, den hinteren Fußbereich sowie einen Teil des Unterschenkels seitlich umfassender, stiefelartiger Teilschuh ausgebildeten Stützteil aus weitgehend starrem Material, wobei in dem Stützteil zumindest im Bereich eines Knöchels eine Ausnehmung und neben der vorderen Öffnung Verschlußelemente vorgesehen sind. Zur Erzielung einer verbesserten Seitenstütz-Stabilität ist bei dieser Stütze der zwischen der Vorderkante und der Ausnehmung für den Knöchel liegende Bereich des Teilschuhs unterbrochen. Des weiteren weist dieser vordere Bereich des Teilschuhs zwei Laschen auf. Durch diese in der Stütze vorgesehene Unterbrechung soll sichergestellt werden, daß insbesondere beim Gehen mit der Stütze auftretende Druck- und Zugbelastungen des vorderen Teils der Stütze abgebaut und somit Ermüdungsbrüche vermieden werden.

Bei den bekannten Stützen für den Sprunggelenkbereich wird zum Teil von stiefelartigen Teilschuhen, von elastischen Knöchelsocken oder schlauchförmigen Bandagen ausgegangen, die zur seitlichen Abstützung des Sprunggelenks Polstereinlagen, Stützstreifen, Steigbügeleinsätze oder besonders ausgestaltete Stützbügel aufweisen, um das Sprunggelenk seitlich zu stützen und zu stabilisieren. Keine der bekannten Stützen bzw. Gelenkbandagen ist so ausgebildet, daß eine tiefwirkende Querfriktion auf den Ansatz der Achillessehne erreicht wird. Eine Kombination einer Friktionsmassage mit einer Gelenkweichteilmassage wird mit keiner der bekannten Stützen oder Bandagen erreicht.

Der Erfindung liegt die Aufgabe zugrunde, eine Sprunggelenk-Bandage der eingangs genannten Art zu schaffen, die das Sprunggelenk begrenzt seitlich abstützt und stabilisierend auf den Kapsel-Band-Apparat einwirkt, und zwar bei gleichzeitiger Abpolsterung des kritischen Bereichs der Bisgaard'schen Kulisse, um eine gleichmäßige Druckverteilung zu erzielen, wobei eine durch Bewegung hervorgerufene intermittierende Kompression auf die Gelenkweichteile um den medialen und lateralen Malleolus und eine tiefe Querfrikton auf den Ansatz der Achillesferse ausgeübt wird.

Diese Aufgabe wird durch die in den Patentansprüchen 1 und 2 gekennzeichneten Merkmale gelöst.

Eine derart erfindungsgemäß ausgebildete Sprunggelenk-Bandage wird eingesetzt bei

- posttraumatischen Reizzuständen und Überlastungserscheinungen des Sprunggelenks,
- Gelenkergüssen und Schwellungen bei Arthrose und Arthritis,
- Achillodynie,
- Myotendopathien,
- Bänderschwäche,
- nach Verletzungen und Ruhigstellung,
- temporär postoperativ.

Diese Sprunggelenk-Bandage stützt das Sprunggelenk seitlich ab und wirkt stabilisierend auf den Kapsel-Band-Apparat ein. Der kritische Bereich der Bisgaard'schen Kulisse wird dabei abgepolstert, um eine gleichmäßige Druckverteilung zu erzielen. Mit der Bandage wird eine intermittierende Kompression auf die Gelenkweichteile um den medialen und lateralen Malleolus ausgeübt. Außerdem bewirkt die Bandage im angelegten Zustand einen seitlichen Druck auf den Bereich, wo die Achillessehne frei im Sehnenschlauch liegt, wobei jedoch die Achillessehne selbst freigelassen wird. Dadurch, daß in dem Schlauchkörper der Sprunggelenk-Bandage zwei über den paraachillären Weichteilen liegende und am medialen und lateralen Malleolus den Bereich über der Bisgaard'schen Kulisse abdeckende Druckpelotten vorgesehen sind,von denen jede Druckpelotte als Formkörper mit einer seitlichen, abgerundeten Aussparung zur Aufnahme des Knöchelvorsprunges aus einem weichen oder weich-elastischen Material ausgebildet ist, in dem ein stabförmiger Friktionskern aus einem harten, inkompressiblen Material angeordnet und in dem Material des Formkörpers in seiner Lage fixiert ist, wird vermittels dieser Friktionskerne eine tiefe Querfriktion auf den Ansatz der Achillessehne ausgeübt. Die Bandage selbst behindert in keiner Weise den venösen Abfluß über den Fußrücken; sie läßt sich problemlos in einem normalen Straßen- oder Sportschuh tragen. Das Gewebe, aus dem der Schlauchkörper der Sprunggelenk-Bandage ausgebildet ist, ist schweißabsorbierend und läßt sich gut waschen.

Bei der Sprunggelenk-Bandage handelt es sich um eine anatomisch geformte Bandage für das Sprunggelenk mit einer rückwärtigen Naht. Die Bandage selbst umfaßt den halben Fuß und reicht bis zur Wade. Im Bereich der Laufsohle ist eine halbe Ferse eingestrickt. Über den paraachillären Weichteilen befinden sich die beiden anatomisch asymmetrisch angeordneten Druckpelotten, die am medialen und lateralen Malleolus den Bereich über der Bisgaard'schen Kulisse abdecken. In ihrem vorderen Abschnitt laufen die Druckpelotten flach aus; an der hinteren Kante fallen sie steil ab. Über die Strecke, wo die Achillessehne frei im Sehnenschlauch läuft, sind die Friktionskerne in das Material des Formkörpers der Druckpelotten unverschiebbar eingegossen bzw. befestigt. Es sind zwei spiegelgleiche Ausführungen für das rechte und das linke Bein vorgesehen.

Durch die Einbeziehung der Friktionsmassage und durch die Kombination aus funktionaler Mobilitätssteigerung und gezielter Schmerzlinderung wird eine sehr effektive Sprunggelenk-Bandage erhalten, die als aktive Friktionsbandage zur Therapie schmerzhafter Reizzustände des Sprunggelenks sowie bei Achillodynie eingesetzt wird. Die Bandage stützt das Sprunggelenk und die Achillessehne und übt bei gleichzeitiger Friktionsmassage eine intermittierende Kompression auf die Gelenkweichteile aus. Ihre besondere Wirkung entfaltet die Sprunggelenk-Bandage vor allem in der Bewegung. Durch die schmerzlose Dauermassage werden Ödeme und Schwellungen rasch abgebaut. Die völlig neuartigen Zwei-Komponenten-Pelotten bewirken eine gezielte seitliche Friktion auf den Ansatz der Achillessehne. Selbst bei akuten Reizungen der Achillessehne erfolgt schon nach kurzer Anwendung eine spürbare Linderung und Funktionsverbesserung.

Die mit der Sprunggelenk-Bandage erreichte Wirkung basiert auf den Faktoren Entlastung, Kompression und Friktionsmassage, wobei die Bandage selbst das Gelenk führt, polstert und begrenzt stabilisiert. Aufgrund der in den Druckpelotten der Bandage angeordneten harten Friktionskerne ist neben einer Massage der Gelenkweichteile durch das Pelottenmaterial eine gezielte Friktionsmassage auf spezielle Schmerzpunkte möglich. Bei dieser Massageform wird der Ansatz der Achillessehne quer zum Ansatz behandelt, wobei die Querfriktion streng lokal angesetzt wird. Diese Friktionsmassage wirkt in akuten Fällen einer Adhäsionsbildung mit angrenzenden Strukturen entgegen und löst in subakuten oder chronischen Fällen bestehende Adhäsionen. Gleichzeitig beseitigt die Friktionsmassage durch die Freisetzung von Histamin und Serotonin aus zerstörten Mastzellen lokale Entzündungsreaktionen biochemisch und führt somit zu einer Schmerzlinderung. Zusätzlich bewirkt der Friktionskern eine laterale Kompression auf die im Falle einer Achillodynie schmerzhaft geschwollene Achillessehne. Die ideale orthometrische Form wird der anatomischen Form des Sprunggelenks angepaßt, so daß eine Bandage für das Sprunggelenk erhalten wird, die bevorzugterweise folgenden Aufbau aufweist:

- Dreidimensional anatomische Formstrickung in Anpassung an das Gelenk,
- Kompressionsdruck 15 mm Hg,
- Zweizugelastik und damit ausgeglichene Druckverteilung in beiden Richtungen,
- besonders ausgeführte Abschlüsse der Bandagenränder mit Druckverminderungsrand, so daß keine Stauungsbeschwerden auftreten,
- viskoselastische Profileinlagen aus Silikon als Aktivelemente; Knochenvorsprünge und Sehnenansätze bestimmen die Form der Pelotte,
- Zwei-Komponentenpelotte mit einem festen, harten Friktionskern zur gezielten Friktionsmassage der kritischen Punkte (Achillessehnenansätze),
- Einsätze aus hochelastischem Relief-Gestrick, in deren Bereich das Material nach dem Faltenbalg-Prinzip wellenförmig gerafft wird. Die Wellen erlauben dabei eine hohe Beweglichkeit; sie nehmen in der Gelenkbeugung das überschüssige Material auf und verhindern eine Faltenbildung,

- hautfreundliches Gewebe aus z.B. vernetztem Polyurethan (Handelsname ELASTAN), Elastodienfasern und Polyamid mit hohem Baumwollanteil, wobei auch Elastofasern eingesetzt werden können, die aufgrund ihres chemischen Aufbaus extrem hoch verformbar sind und die Eigenschaft aufweisen, nach Aufheben der Verformungskräfte im wesentlichen augenblicklich und fast vollständig in den ursprünglichen Zustand zurückzukehren, d.h. es sind solche hochelastischen Fasern, die eine hohe elastische Dehnung besitzen. Diese hochelastischen Fasern können aus Gummifäden, aus Kautschuk und anderen synthetischen Elastofasern bestehen, die nicht auf der Basis von Polyurethan hergestellt sind. Bei den Elastodienfasern handelt es sich um Fasern aus natürlichem Polyisopren (Gummi) oder synthetischem Polyisopren oder aus solchen Polymeren, die durch Polymerisation eines oder mehrerer Diene, evtl. unter Zusatz eines oder mehrerer Vinylmonomere, entstehen.

Nach einer weiteren Ausführungsform der Erfindung ist der Schlauchkörper der Sprunggelenk-Bandage mit einem zwischen dem die Laufsohle bildenden Abschnitt und dem oberen Schlauchkörperabschnitt liegenden, zwickelförmigen Einsatz aus einem hochelastischen Relief-Gestrick versehen. Insbesondere bei Sprunggelenk-Bandagen tritt aufgrund des dort gegebenen Beugungswinkels häufig das Problem unerwünschter Faltenbildung auf. Diesem Problem kann dadurch entgegengewirkt werden, daß die Bandage zumindest in den kritischen Beugungsbereichen mit einem höher elastischen Garn gestrickt wird. Derartige Garne sind allerdings verhältnismäßig teuer. Darüber hinaus führt die Verwendung dieser Garne dazu, daß sich im Streck- bzw. Ruhezustand des Gelenks eine zu hohe Kompression einstellt, die zu Abschnürungen der Blutgefäße führen kann und dementsprechend beim Tragen der Bandage Unannehmlichkeiten verursacht. Aus diesem Grunde ist ein aus Textilfäden hergestelltes Flächengebilde zur Verwendung im inneren und äußeren Beugebereich einer Sprunggelenk-Bandage geschaffen, das wirtschaftlich herstellbar ist und mit dem zuverlässig ausgeschlossen werden kann, daß sich im Beugebereich der Sprunggelenk-Bandage selbst nach längerem Gebrauch Falten bilden, so daß ein besserer Tragekomfort der Bandage erzielt wird.

Durch die Merkmale des Patentanspruches 22 wird ein aus Textilfäden bestehendes Flächengebilde geschaffen, das so aufgebaut ist, daß zumindest auf einer Seite ein Relief in Form einer Wellenstruktur entsteht. Diese Wellenstruktur wird durch eine darunterliegende Fadenanordnung aus höherelastischem Garn bzw. Faden elastisch vorgespannt und stabilisiert, wodurch die Deckstruktur im entspannten Zustand des Flächengebildes wellenartig, vorzugsweise halbwellenartig, ausbaucht. Wenn dementsprechend die Flächenstruktur bei Gelenkbandagen eingesetzt und senkrecht zur Ausrichtung der Querwellen beansprucht wird, was beispielsweise beim Beugen des bandagierten Gelenks der Fall ist, so werden zunächst lediglich die Querwellen flacher bzw. glatt gezogen, ohne daß eine Streckung bzw. Reckung der Deckstruktur erfolgt. Überdehnungen der Deckstruktur können auf diese Art und Weise zuverlässig ausgeschlossen werden, wodurch gleichzeitig das Auftreten von Falten, und zwar selbst nach längerem Gebrauch der Bandage, vermieden wird. Dieses Flächengebilde eignet sich deshalb insbesondere zur Verwendung in Sprunggelenk-Bandagen, da das Gelenk einen großen Bewegungsfreiraum bzw. Beugewinkel aufweist.

Der Aufbau des textilen Flächengebildes erlaubt somit trotz verhältnismäßig hohen Dehnungsvermögens die Verwendung von verhältnismäßig unelastischem Garn für die Deckstruktur, wodurch die Voraussetzungen für eine wirtschaftliche Herstellung des textilen Flächengebildes geschaffen werden.

Besonders vorteilhaft läßt sich das aus Textilienfäden hergestellte Flächengebilde mit den oben dargelegten Eigenschaften als Gestrick ausbilden, da hierdurch bereits eine große Grund-Elastizität des textilen Flächengebildes bereitgestellt wird. Die Gefahr von Überdehnungen des Garns kann mit dieser Ausführung zusätzlich vermindert werden.

Vorzugsweise wird das textile Flächengebilde in der Sprunggelenk-Bandage nur bereichsweise eingesetzt, nämlich dort, wo die größten Dehnungswege zu erwarten sind. Die Ausrichtung der Querwellen erfolgt regelmäßig senkrecht zur Haupt-Dehnungsrichtung. Es ist auch möglich, in dem durch die Gelenkbeugung höchst beanspruchten Bereich der Sprunggelenk-Bandage mehrere textile Flächenstrukturen so anzuordnen, daß die jeweiligen Querwellen-Ausrichtungen im Winkel zueinander stehen.

Weitere vorteilhafte Ausgestaltungen der Erfindung sind in den Unteransprüchen gekennzeichnet.

Ausführungsbeispiele der Erfindung werden nachstehend anhand der Zeichnung näher erläutert. Es zeigen

Fig. 1 von der medialen Seite her gesehen eine Sprunggelenk-Bandage in einer Seitenansicht mit an dieser angeordneten Druckpelotte und mit einem im Gelenkbewegungsbereich angeordneten Einsatz aus einem Relief-Gestrick,

Fig. 2 von der lateralen Seite her gesehen die Sprunggelenk-Bandage gemäß Fig.1 in einer Seitenansicht,

Fig. 3 in einer Ansicht von oben die einseitig aufgeschnittene und aufgefaltete Sprunggelenk-Bandage mit den beiden in den Bandagenseitenteilen angeordneten Druckpelotten,

Fig. 4 in einer Seitenansicht einen Fuß mit dem Sprungbein,

Fig. 5 den menschlichen Fuß in medialer und lateraler Ansicht,

Fig. 6 eine Rückansicht des Unterschenkels mit der Achillessehne eines menschlichen Beines

Fig. 7 von der medialen Seite her gesehen eine Druckpelotte in Seitenansicht mit eingesetztem Friktionskern,

Fig. 8 von der lateralen Seite her gesehen eine Druckpelotte in Seitenansicht mit in dieser angeordnetem Friktionskern,

Fig. 9 in einer Seitenansicht eine Druckpelotte in verwendungsfähiger Größe,

Fig.10 einen senkrechten Längsschnitt gemäß Linie X-X in Fig.9,

Fig.11 einen Längsschnitt gemäß Linie XI-XI in Fig.9,

Fig.12 in einer schaubildlichen Ansicht einen Friktionskern,

Fig.13 einen senkrechten Schnitt gemäß Linie XIII-XIII in Fig.12,

Fig.14 und 15 die verschiedenen Phasen des Anlegens der Sprunggelenk-Bandage am Fuß,

Fig.16 einen vergrößerten Schnitt durch den Einsatz aus dem Relief-Gestrick gemäß Linie XVI-XVI in Fig.15,

Fig.17 in schematisch vereinfachter Darstellung das Prinzip der Verformung des aus textilen Fäden hergestellten und den Einsatz in der Sprunggelenk-Bandage bildenden Flächengebildes,

Fig.18 Darstellungen von Strickreihen zur Verdeutlichung einer ersten Ausführungsform für ein Verfahren zur Herstellung des Relief-Gestricks, und

Fig.19 in einer der Fig.18 ähnlichen Darstellung ein weiteres Strickmuster für das Relief-Gestrick.

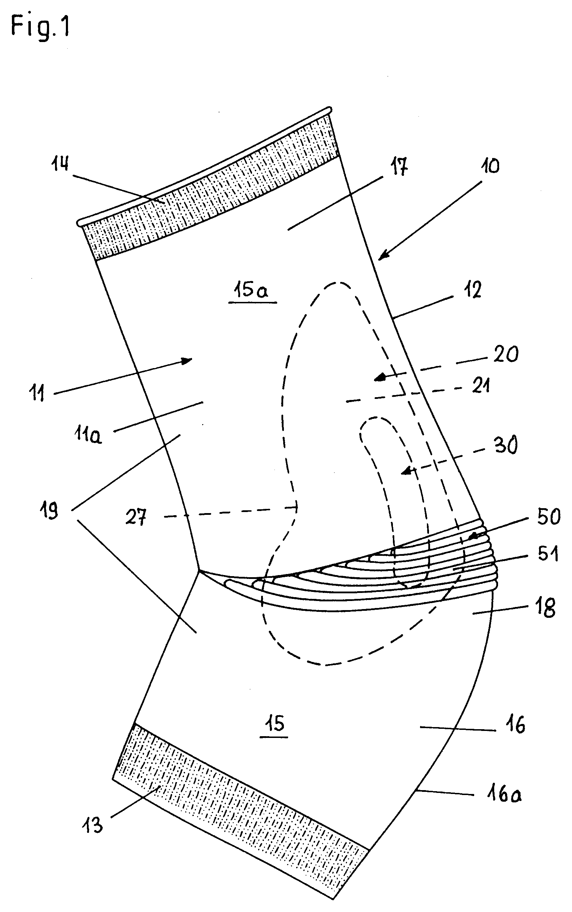

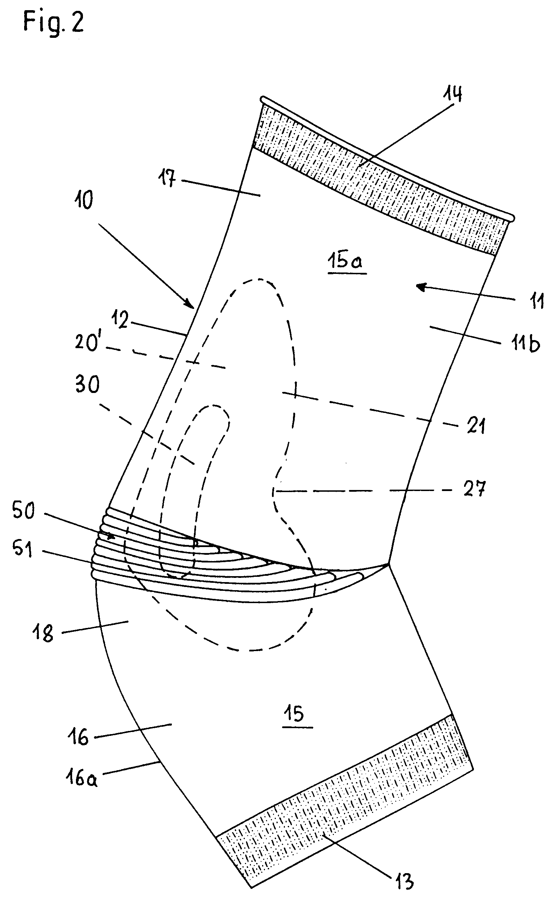

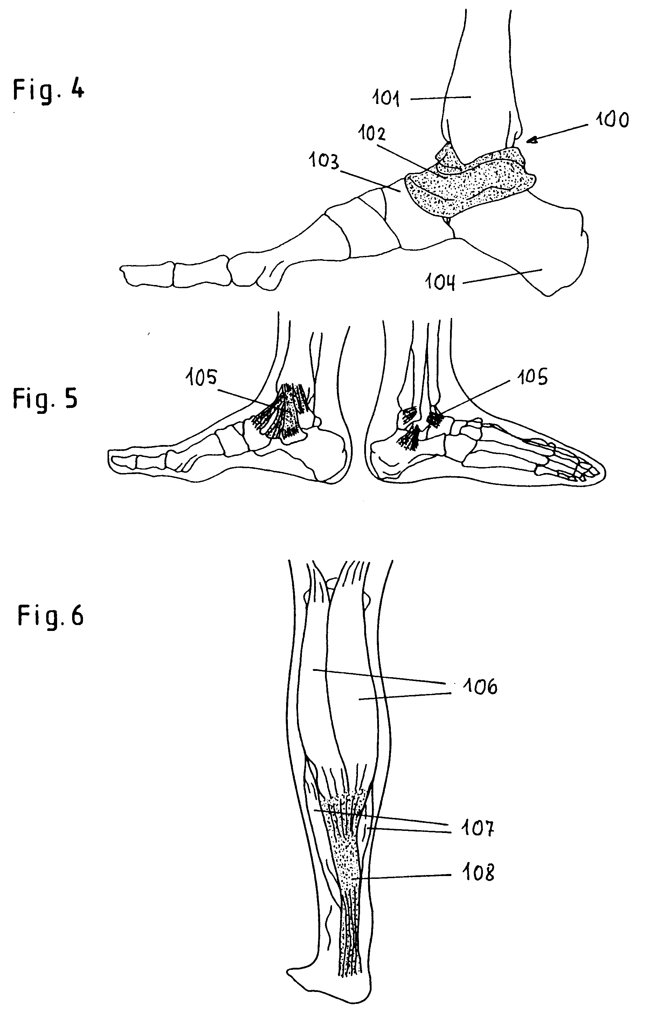

Die in Fig. 1 und 2 dargestellte Sprunggelenk-Bandage 10 besteht aus einem anatomisch geformten Schlauchkörper 11 aus einem Gewebe oder Gestrick. Dieser Schlauchkörper 11 weist eine Länge auf, aufgrund der die Sprunggelenk-Bandage 10 mit einem längeren Abschnitt 17 oberhalb des Gelenkes und mit einem die Laufsohle 16a bildenden kürzeren Abschnitt 16 unterhalb des Gelenks endet. Im entfalteten Zustand weist der Schlauchkörper 11 die aus Fig.3 ersichtliche Zuschnittsform auf, wobei die beiden den Schlauchkörper 11 bildenden Seitenteile 11a,11b über eine Längsnaht 12 miteinander verbunden sind, wenn beide Schlauchkörperseitenteile 11a,11b zum Schlauchkörper 11 gebildet sind (Fig.1 und 2). Der Schlauchkörper 11 kann auch rundgestrickt sein.Der untere Rand des Schlauchkörpers 11 ist mit 13 und der obere Rand mit 14 bezeichnet.

Die Sprunggelenk-Bandage 10 weist somit einen kürzeren unteren Schenkel 15 und einen oberen längeren Schenkel 15a auf (Fig.1 und 2). Im Verbindungsbereich der beiden Schenkel 15,15a ist der Schlauchkörper 11 der Sprunggelenk-Bandage 10 mit einem keilförmigen Einsatz 50 aus einem hochelastischen Relief-Gestrick 51 versehen, wobei im angelegten Zustand der Sprunggelenk-Bandage 10 der Schlauchkörper 11 dann so angeordnet ist, daß der Einsatz 50 aus dem hochelastischen Relief-Gestrick 51 im Beugungsbereich des Fußgelenkes zu liegen kommt.

Der in Fig.4 dargestellte menschliche Fuß 100 läßt die Anordnung des Schienbeins 101, des Sprungbeins 102, des Kahnbeins 103 und des Fersenbeins 104 erkennen. Das obere Sprunggelenk erlaubt als Scharniergelenk Streck- und Beugebewegungen. Das Schienbein und das Wadenbein umfassen zangenförmig die Gelenkrolle des Sprungbeins 102 und bilden die Malleolengabel. Zusätzliche Stabilität wird erhalten durch das innere und äußere Kollateralband. Die Bänder 105 sind in ihrer Vorlaufstruktur gefächert, so daß bei jeder Gelenkstellung jeweils ein Teil angespannt ist (Fig.5). Fig.6 zeigt den Unterschenkel eines menschlichen Beines mit den wesentlichen Muskeln und der Achillessehne 108. Mit 106 ist der Zwillingsmuskel und mit 107 der Schollenmuskel bezeichnet.

Der Schlauchkörper 11 der Sprunggelenk-Bandage 10 weist eine halbe Ferse 18 auf, die im Bereich der Laufsohle des Schlauchkörperabschnittes 16 eingestrickt ist. Über dieser halben Ferse 18 ist der keilförmige Einsatz 50 eingearbeitet (Fig.1 und 2).

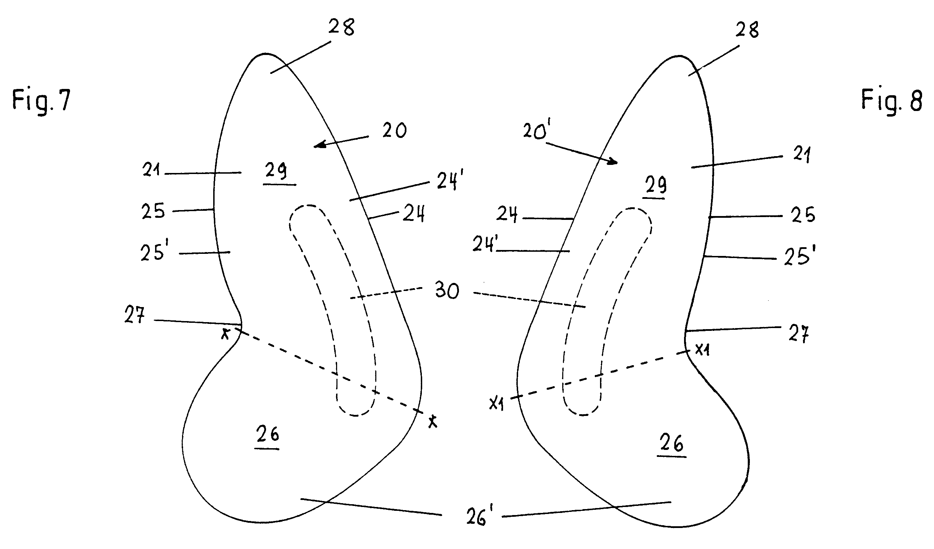

In jedes Seitenteil 11a,11b des Schlauchkörpers 11 der Sprunggelenk-Bandage 10 ist eine Druckpelotte 20 bzw. 20' eingearbeitet. Im angelegten Zustand der Sprunggelenk-Bandage 10 befinden sich diese beiden anatomisch asymmetrisch ausgebildeten Druckpelotten 20,20' über den paraachillären Weichteilen. Jede Druckpelotte 20,20' ist mit einer seitlichen Aussparung 27 versehen (Fig.7 und 8). Ein richtiger Sitz der Sprunggelenk-Bandage 10 ist dann gegeben, wenn die Knöchelvorsprünge in den abgerundeten Aussparungen 27 der Druckpelotten 20,20' liegen. Im angelegten Zustand der Sprunggelenk-Bandage 10 umfaßt deren Schlauchkörper 11 den halben Fuß; der Schlauchkörper reicht mit seinem anderen Ende bis zur Wade. Ist die Sprunggelenk-Bandage 10 richtig angelegt, dann beaufschlagen die Druckpelotten 20,20' das Gelenk derart, daß ein Kompressionsdruck auf die Gelenkweichteile und Sehnenansätze und eine gezielte Friktionsmassage auf spezielle Schmerzpunkt ausgeübt wird. Für die Befestigung der Druckpelotten 20,20' an der Bandage 10 ist diese im Befestigungsbereich bevorzugterweise doppellagig ausgebildet, wobei dann in dem durch diese Doppellagigkeit ausgebildeten Zwischenraum die Druckpelotten 20,20' angeordnet sind, die die in Fig.7,8 und 9 dargestellte Form aufweisen. Jedoch auch andere bekannte Befestigungsformen für die Druckpelotten, z.B. wie Schweiß-, Klebe- oder Nähverbindungen, sind möglich. Beide Druckpelotten 20,20' weisen gleiche Formgebungen und gleiche Abmessungen auf, worauf nachstehend noch näher eingegangen wird.

Jede Druckpelotte 20,20' ist als Formkörper 21 ausgebildet und weist eine durch die Knochenvorsprünge und Sehnenansätze des Gelenks vorgegebene Form auf. Die Formkörper 21 der Druckpelotten 20,20' bestehen aus einem weich-elastischen Material. Beide Druckpelotten 20,20' sind den Knochenvorsprüngen auf der medialen Seite und der lateralen Seite entsprechend ausgebildet.

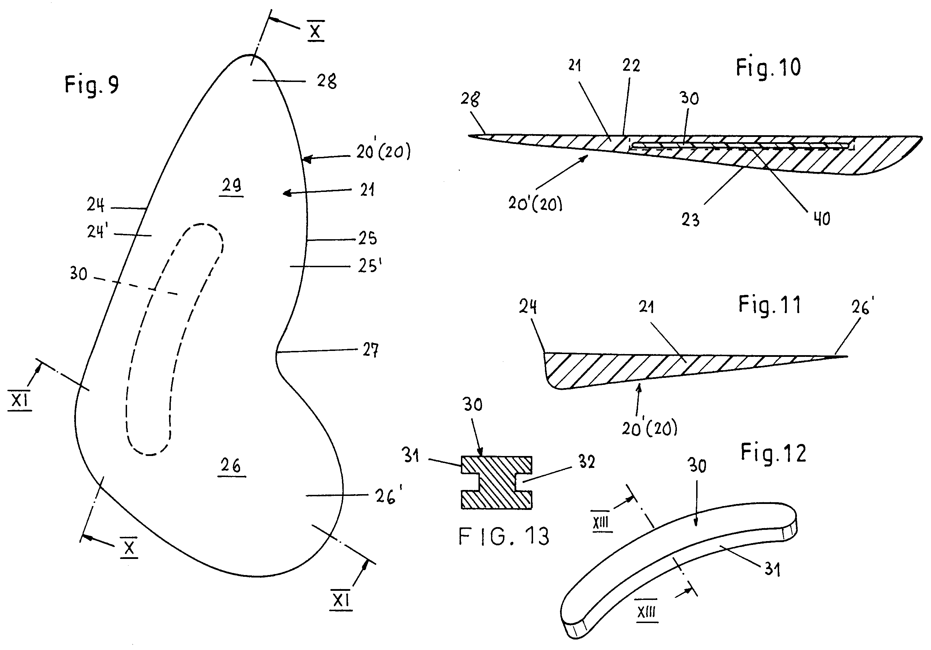

In dem Material des Formkörpers 21 einer jeden Druckpelotte 20,20' ist ein fester Friktionskern 30 fixiert, der aus einem harten Material, wie z.B. einem inkompressiblen Polyamid, Polyurethan, Silikonkautschuk,oder einem Material mit gleichen Elastizitätseigenschaften oder einem unelastischen Material besteht. Dieser Friktionskern 30 einer jeden Druckpelotte 20,20' ist stab- und bogenförmig ausgebildet (Fig.12). Seine Anordnung in dem Formkörper 21 der Druckpelotte 20 bzw. 20' erfolgt benachbart zur äußeren Formkörperlängskante 24 des Formkörpers 21, und zwar im Übergangsbereich des längeren Formkörperabschnittes 29 zum kürzeren Formkörperabschnitt 26 bei einer etwa L-förmigen Ausgestaltung der Druckpelotte 20,20'.

Der Friktionskern 30 mit einem kreisförmigen, quadratischen, rechteckförmigen oder eine andere geometrische Form aufweisenden Querschnitt weist im Bereich seiner umlaufenden Wandfläche 31 eine Einziehung in Form einer Nut 32, rillenförmige Ausnehmungen, Hinterschneidungen, Verzahnungen od. dgl. auf, die zur Aufnahme des Formkörpermaterials dient, damit der Friktionskern 30 in dem Formkörper 21 der Druckpelotte 20 bzw. 20' in seiner Lage fixiert ist. Bevorzugterweise ist die Anordnung des Friktionskerns 30 in dem Formkörper 21 so getroffen, daß dieser dicht unterhalb der Oberfläche des Formkörpers 21 zu liegen kommt, um so einen hohen Druck auf die Gelenke ausüben zu können (Fig.10 und 13).

Der Formkörper 21 besteht aus einem weichen oder weichelastischen Material. Dagegen besteht der Friktionskern 30 aus einem harten oder inkompressiblen Material; er ist in dem Material des Formkörpers 21 in seiner Lage fixiert. Der Friktionskern 30 weist gegenüber der Härte des Formkörpers 21 eine größere Härte auf. Die Differenz zwischen der Härte des Formkörpers 21 und der Härte des Friktionskerns 30 beträgt mindestens 10 Shore A, vorzugsweise 20 Shore A.

Die Härte des Materials, aus dem der Formkörper 21 besteht, liegt unterhalb 50 Shore A, wohingegen die Härte des Materials, aus dem der Friktionskern 30 gefertigt ist, oberhalb 50 Shore A liegt, worauf nachstehend noch näher eingegangen wird.

Die Druckpelotte 20 bzw. 20' weist bevorzugterweise die in Fig. 9 bis 11 dargestellte Form auf. Hiernach ist der Formkörper 21 mit einer flachen Grundfläche 22 und einer der Grundfläche abgewandten Außenwölbung 23 versehen. Der Formkörper 21 der Druckpelotte 20 bzw. 20'ist etwa L-förmig mit einem kurzen, halbkreisförmig auslaufenden Abschnitt 26' und mit einem unter Ausbildung der inneren abgerundeten, im Bereich der inneren Längskante 25 liegenden Aussparung 27 längeren, in einen etwa keilförmigen Bereich 28 auslaufenden Abschnitt 29 ausgebildet. In seinem vorderen Bereich 25' läuft der Formkörper 21 flach aus. An seiner hinteren Längskante 24 fällt dagegen der Formkörper 21 ab. Anstelle einer flachen Grundfläche 22 kann der Formkörper 21 auch eine profilierte oder leicht nach innen eingezogene Grundfläche aufweisen. Die Anordnung der beiden Druckpelotten 20,20' an den Seitenteilen 11a,11b des Schlauchkörpers 11 ist dabei derart, daß die Formkörper 21 der beiden Druckpelotten 20,20' mit ihren flachen Grundflächen 22 außenliegend sind. Die Außenwölbungen 23 der Druckpelotten 20, 20' sind dagegen einander zugekehrt. Beide Druckpelotten 20,20' sind mit ihren äußeren Längskanten 24 benachbart zur Längsnaht 12 des Schlauchkörpers 11 an bzw. in den Seitenteilen 11a,11b angeordnet. Die Friktionskerne 30 der beiden Druckpelotten 20,20' sind dabei zu den äußeren Längskanten 24 der Formkörper 21 der beiden Druckpelotten 20,20' im Übergangsbereich des langen Formkörperabschnittes 29 zum kürzeren Formkörperabschnitt 26 angeordnet.

Der Formkörper 21 besteht aus einem weichen oder weichelastischen Material, wie Filz, Moosgummi, Neopren,Gummi oder aus viskoelastischem Silikonkautschuk oder aus einem elastischen, kompressiblen, durch Druck verformbaren Silikonkautschuk z.B. mit einer Härte von 40 Shore A, einem Silikonschaum mit einer Härte von 9 bis 13 Shore A oder einem kompressiblen, durch Druck verformbaren und ohne Sprungelastizität in seine Form sich rückbildenden Silikonkautschuk vom Typ eines nach dem Verfahren der Polyaddition vulkanisierenden Kaltkautschuks, der neben einer hohen Flexibilität eine unter 4 Shore A liegende Härte aufweist, wobei jedoch auch Silikonkautschuke eingesetzt werden können, deren Härte oberhalb von 4 Shore A liegt. Derartige viskoelastische Silikonkautschuke oder Materialien mit gleichen Elastizitätseigenschaften besitzen die Eigenschaft, bei angelegter Bandage mit einer derartigen Druckpelotte aufgrund der gleitenden Bewegung, ausgelöst durch Masseverschiebung, bei Druckanwendung oder bei Bewegungsabläufen im Auflagebereich massierend zu wirken. Für die Herstellung des Formkörpers 21 sollte ein Material gewählt werden, welches viskoelastisch ist und aufgrund seiner elastischen Eigenschaften eine Massage bewirkt.

Der Friktionskern 30 der Druckpelotte 20 bzw. 20' besteht dagegen aus einem harten oder inkompressiblen Kunststoff mit z.B. einer über 50 Shore A liegenden Härte und weist gegenüber dem Formkörper 21 eine weitaus größere Härte auf, damit in der Bewegung eine gezielte Friktionsmassage auf spezielle Schmerzpunkte der Gelenke erreicht wird. Als Material für den Friktionskern kommt natürlicher oder künstlicher Kautschuk oder Hartgummi infrage. So kann u.a. ein Chloropren-Polymerisat (Handelsname NEOPREN) mit einer Härte von 50 Shore A, ein gummielastisches, vernetztes Polyurethan (Handelsname VULKOLLAN) mit einer Härte von 65 bis 90 Shore A, ein Silikonkautschuk mit einer Härte von 60 Shore A, ein Ethylen-Propylen-Dien-Kautschuk (EPDM) mit einer Härte von 80 Shore A oder ein Copolymerisat mit Acrylnitril (Handelsname PERBUNAN) mit einer Härte von 70 Shore A, ferner ein Polyamid verwendet werden. Jedoch auch andere Kunststoffe oder Naturstoffabkömmlinge können zur Herstellung des Friktionskerns 30 herangezogen werden. Wesentlich ist, daß der Friktionskern 30 eine ausreichende Härte aufweist, um die gezielte Friktionsmassage auf spezielle Schmerzpunkte ausüben zu können. Der Friktionskern 30 kann auch aus Metall oder Holz bestehen.

Der Friktionskern 30 besteht aus einem Material, das gegenüber dem Material der Druckpelotte etwas härter ist. So besteht auch die Möglichkeit, für die Druckpelotte ein Material mit einer Härte von 4 Shore A und für den Friktionskern 30 ein Material mit einer Härte von z.B. 15 Shore A zu verwenden. Bei angelegter Bandage liegen die Friktionskerne 30 der beiden Druckpelotten 20,20' auf dem Ansatz der Achillessehne, so daß eine gezielte seitliche Friktion auf diesen Ansatz der Achillessehne bewirkt wird, wohingegen die Druckpelotten 20,20' auf den Gelenkweichteilen aufliegen, auf die eine intermittierende Kompression ausgeübt wird.

Nach einer weiteren Ausführungsform der Erfindung ist der Friktionskern 30 in der Druckpelotte 20 bzw. 20' auswechselbar angeordnet. Hierzu ist der Formkörper 21 mit einer Ausnehmung 40 von etwa der Größe des Friktionskerns 30 versehen, in die der Friktionskern 30 vermittels eines leichten Druckes eingedrückt wird (Fig.10). Die die Ausnehmung in der Druckpelotte 20 bzw. 20' begrenzende Innenwandfläche weist eine Profilgebung auf, die einen Eingriff in das Profil der umlaufenden Wandfläche des Friktionskerns 30 ermöglicht und da das Druckpelottenmaterial elastisch ist, der Friktionskern 30 jedoch gegenüber dem Druckpelottenmaterial eine größere Härte aufweist, läßt sich der Friktionskern 30 in die Ausnehmung pressen, wobei während des Preßvorganges das Profil der Innenwandfläche so zusammengepreßt wird, daß der Friktionskern 30 gänzlich in die Ausnehmung 40 gleiten kann und aufgrund des Rückstellvermögens des Materials der Druckpelotte 20 wird das Pelottenmaterial in das Randprofil des Friktionskerns 30 gepreßt, so daß dieser fest in der Druckpelotte gehalten wird. Durch entsprechende Verformung der Druckpelotte durch eine starke äußere Druckanwendung kann der Friktionskern 30 aus der Druckpelotte herausgedrückt werden. Dadurch ist die Möglichkeit gegeben, Friktionskerne unterschiedlicher Härte verwenden zu können. In dem Fall, in dem Druckpelotten 20 bzw. 20' mit auswechselbarem Friktionskern 30 verwendet werden, ist die Druckpelotte an der Bandage derart befestigt, daß ein Abnehmen der Druckpelotte möglich ist.

Der Friktionskern 30 kann als Formkörper ausgebildet sein; er ist dann in dem Material des Formkörpers 21 angeordnet. Nach einer weiteren Ausführungsform der Erfindung ist das Material des aus einem Kunststoff, z.B. Silikonkautschuk, bestehenden Friktionskerns 30 mit dem Material des Formkörpers 21 verschmolzen und mit dem Formkörper 21 unlösbar verbunden. Der Friktionskern 30 kann auch bei der Herstellung des Formkörpers 21 durch Materialaushärtung eines Abschnittes, der den späteren Friktionskern bilden soll und daher eine größere Härte gegenüber dem weichen Material des Formkörpers 21 aufweist, des Formkörpers 21 erhalten werden. In beiden Fällen sollte als Material bevorzugterweise Silikonkautschuk verwendet werden.

Des weiteren kann der Formkörper 21 auch beutelförmig ausgebildet sein. Dieser Beutel besteht aus weich-elastischen Kunststoffen. Der Innenraum des Beutels ist mit einem gasförmigen Medium, wie z.B. Luft, oder einem flüssigen Medium, wie z.B. einem zähflüssigen Silikonöl, Wasser od.dgl. gefüllt. Der Friktionskern 30 ist dann an der Innenwandfläche des Beutels in seiner Lage fixiert.

Die Länge der Sprunggelenk-Bandage 10 beträgt bevorzugterweise gemessen an der Längsnaht 12 vom oberen Rand 14 bis zum keilförmigen Einsatz 50 etwa 17,5 cm und 14 cm vom unteren Rand 13 bis zum Einsatz 50. Die Höhe des oberen Randes 14 beträgt etwa 3,0 cm und die Höhe des unteren Randes 13 des Schlauchkörpers 11 etwa 2,4 cm. Bei diesen Abmessungen der Sprunggelenk-Bandage 10 sollte jede Druckpelotte 20 bzw. 20' eine maximale Breite von etwa 8,5 cm und eine Länge von 15,5 cm haben. Die Position einer jeden Druckpelotte 20 bzw. 20' an dem Schlauchkörper 11 ist derart, daß, und zwar gemessen am oberen hintere Ende, der Abstand vom oberen Rand 14 etwa 6,5 cm bei einem Abstand von der Längsnaht 12 von etwa 0,5 cm beträgt. Die Druckpelotten 20, 20' werden mit ihrer gewölbten Seite nach innen angebracht.

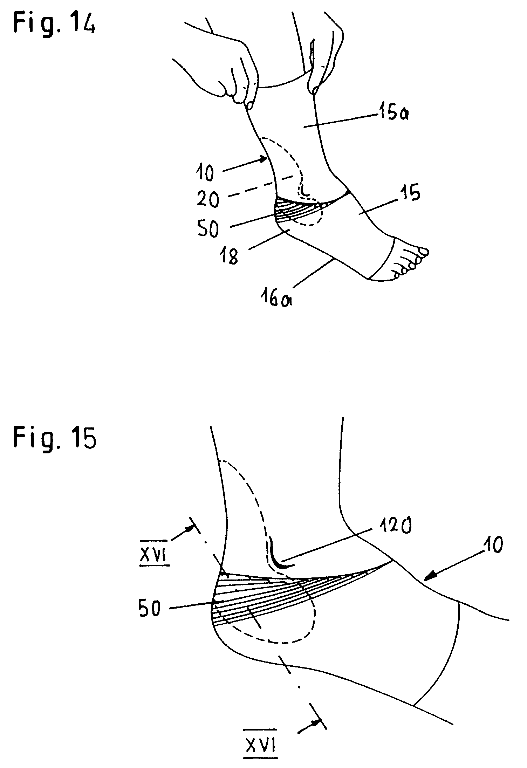

Die Sprunggelenk-Bandage 10 wird gemäß Fig.14 und 15 wie folgt angelegt:
Die Bandage wird zunächst mit der Längsnaht 12 nach hinten am oberen Rand 14 ergriffen und wie ein Strumpf angezogen, wobei der Fuß in Spitzfuß-Position zu halten ist (Fig.14). Die Sprunggelenk-Bandage 10 sitzt richtig, wenn die Knöchelvorsprünge in den seitlichen und gewölbten Aussparungen 27 der Druckpelotten 20,20' liegen, wobei in Fig.15 der Knöchel bei 120 angedeutet ist. Der Einsatz 50 mit dem Relief-Gestrick 51 erlaubt dabei eine hohe Beweglichkeit; er liegt hinten an der Ferse, jedoch nicht an der Fußsohle.

Entsprechend Fig.1 und 2 besteht der Schlauchkörper 11 der Sprunggelenk-Bandage 10 im wesentlichen aus drei Abschnitten, nämlich dem unteren Rand 13, dem oberen Rand 14, um eine rutschfeste Fixierung der Sprunggelenk-Bandage 10 zu gewährleisten, und dem sich an die Ränder 13,14 anschließenden Kompressionsabschnitt 19, der über das Gelenk hinwegläuft (Fig.1). In diesem Kompressionsabschnitt 19 ist der Einsatz 50 aus dem Relief-Gestrick 51 eingearbeitet, und zwar dort, wo aufgrund der Gelenkbeugungen bzw. Bewegungen die größte Dehnung der Sprunggelenk-Bandage 10 auftritt.

Der Schlauchkörper 11 besteht bevorzugterweise aus Strickware, wobei für die einzelnen Abschnitte 13,14,19 und 50 unterschiedliche Strickarten Anwendung finden. Es sind jedoch auch andere Textilwaren und/oder Maschenwaren verwendbar.

Die Besonderheit der gezeigten Sprunggelenk-Bandage besteht darin, daß für den Einsatz 50, der dort vorgesehen ist, wo die höchsten Dehnungs-Wechselbeanspruchungen der Bandage auftreten, eine besondere Textilstruktur, nämlich das Relief-Gestrick 51, verwendet wird, das nachstehend näher erläutert wird.

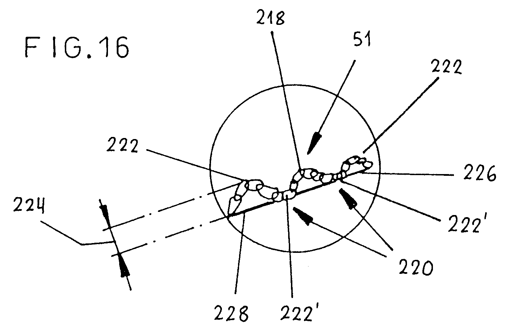

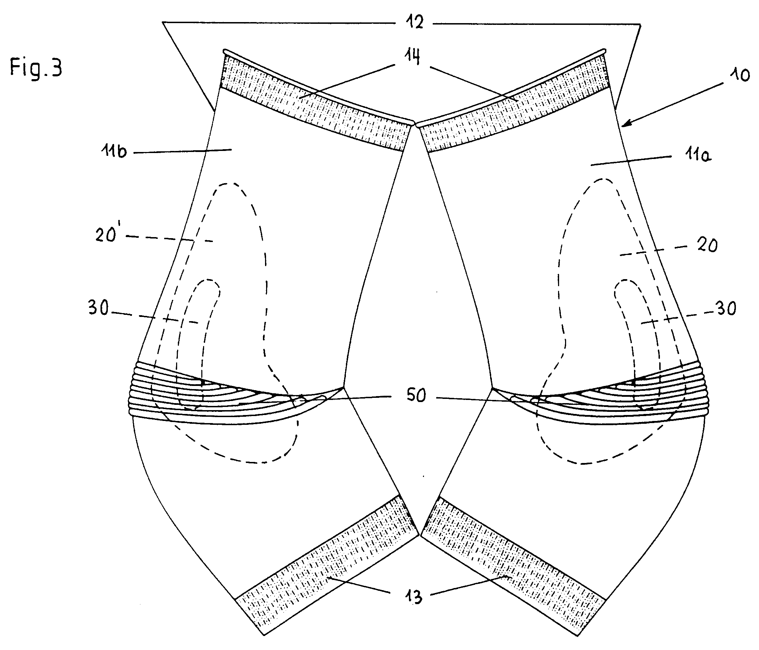

Die streifenförmige Hinterlegung des Einsatzes 50 deutet an, daß das Gestrick in diesem Bereich ein Relief bildet, das aus dem Schnitt XVI-XVI in Fig.15 in der Darstellung gemäß Fig. 16 verdeutlicht wird und mit 51 bezeichnet ist. Bei diesem Relief-Gestrick 51 handelt es sich um eine Wellenstruktur, die zumindest auf einer Seite der Sprunggelenkbandage - bei der gezeigten Ausführungsform auf der dem Körper abgewandten Seite - ausgebildet wird. Dabei ist eine Reihe von Halbwellen unter Zwischenschaltung von Knotenpunkten bzw. Knotenbereichen 220 aneinandergereiht, die wie folgt entstehen:

Ein von Maschen 222 gebildetes Deckgestrick 224 ist im Bereich einzelner Maschen 222' mit einer elastischen Fadenanordnung 226 auf der Unterseite des Deckgestricks 224 derart fest verbunden, daß eine Reihe von Maschen 222 des Deckgestricks 224 - bei der Ausführungsform vier - von einer längeren Masche 228 der darunterliegenden,elastischen Fadenanordnung 226 überbrückt wird. Die Maschen 222 zwischen den Knotenpunkten bzw. - bereichen 220 werden dadurch nach oben ausgebaucht, wodurch die Querwellen 218 vorgespannt und stabilisiert werden. Durch Variation der Anzahl der überbrückten Maschen 222 und/oder der Maschen 222' kann auf das Verformungsverhalten und die Dauerelastizität gezielt Einfluß genommen werden.

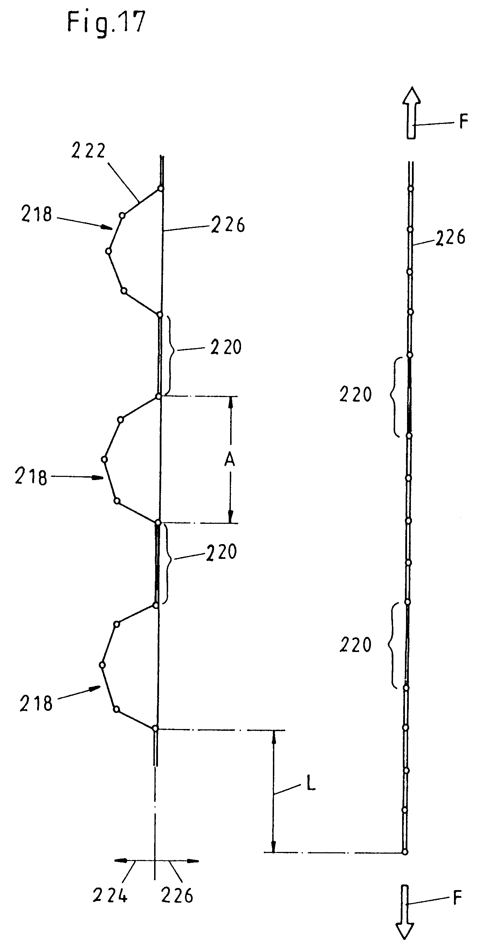

Das Verformungsverhalten des auf diese Art und Weise gebildeten Relief-Gestricks 51 geht im einzelnen aus der Darstellung gemäß Fig. 17 hervor. Auf der linken Seite ist das Gestrick im entlasteten Zustand gezeigt. Die Maschen sind durch Linien angedeutet und die Verbindung der Maschen untereinander durch kleine Kreise.

Das auf der Unterseite vorgesehene elastische Garn 226 überbrückt die Knotenbereiche 220, zwischen denen jeweils vier Maschen 222 des Deckgestricks 224 ausgebildet sind. Durch die Vorspannung der elastischen Fadenanordnung 226 werden die Maschen 222 zu Halbwellen ausgebaucht, wodurch die Reliefstruktur entsteht. Die Halbwellen weisen jeweils zwischen zwei und zwölf, vorzugsweise vier, Maschenreihen auf.

Auf der rechten Seite ist dargestellt, wie sich das Gestrick bei Beanspruchung durch eine Zugkraft F verhält. Man erkennt, daß die elastische Fadenanordnung 226 zwischen den Knotenbereichen 220 gedehnt wird, ohne daß eine zusätzliche Dehnungsbeanspruchung im Bereich der Halbwellen, d.h. im Bereich der Maschen 222, auftritt. Das Gestrick kann sich dementsprechend um das Maß L längen, bevor die Maschen 222 des Deckgestricks 224 auf Zug beansprucht werden. Dieses Maß L schafft dementsprechend eine Dehnungsreserve des Bandagengestricks im Vergleich zu herkömmlichen textilen Flächengebilden.

Die Verbindung zwischen Deckgestrick 224 und elastischer Fadenanordnung 226 kann auf verschiedenste Art und Weise erfolgen. Es ist auch möglich, die Verbindung derart zu wählen bzw. herzustellen, daß auf beiden Seiten des Gestricks Wellen gebildet werden. Das Deckgestrick 224 braucht nicht ausschließlich einflächig ausgebildet zu sein.

Durch die vorstehende Struktur des Gestricks kann trotz Bereitstellung einer hohen Dehnungselastizität für das Deckgestrick normales Strickgarn, wie z.B. Baumwolle oder Polyamidgarn, verwendet werden. Für die elastische Fadenanordnung 226 verwendet man vorzugsweise höherelastisches Garn, wie z.B. Umwindegarn, wobei dieser elastische Faden zusätzlich plattiert werden kann, um die Verschleißfestigkeit des textilen Gewebes zu verbessern.

In die Maschen 222 des Deckgestricks 224 kann ferner ein Einlegefaden eingearbeitet werden, um auch in diesem Bereich der wellenförmigen Reliefstruktur eine Kompressionswirkung der Bandage zu erreichen.

Das auf diese Art und Weise geschaffene Wellengestrick kann auch zusätzlich beispielsweise mit elastischem Garn abgesteppt werden.

Abweichend von den dargestellten Ausführungsbeispielen ist es auch möglich, im Beugungsbereich der Sprunggelenk-Bandage mit mehreren unterschiedlich orientierten Einsätzen 50 zu arbeiten, um auf diese Weise der spezifischen Dehnungsbeanspruchung der Bandage Rechnung zu tragen.

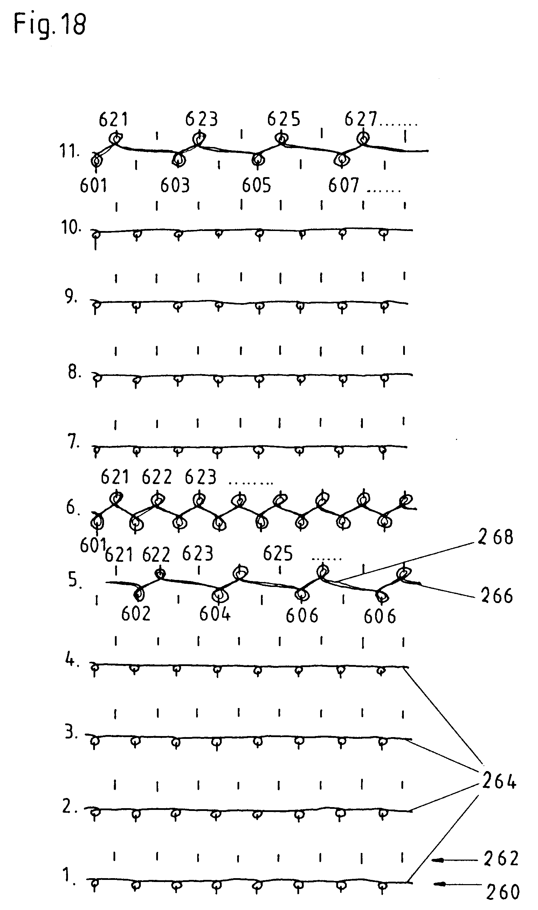

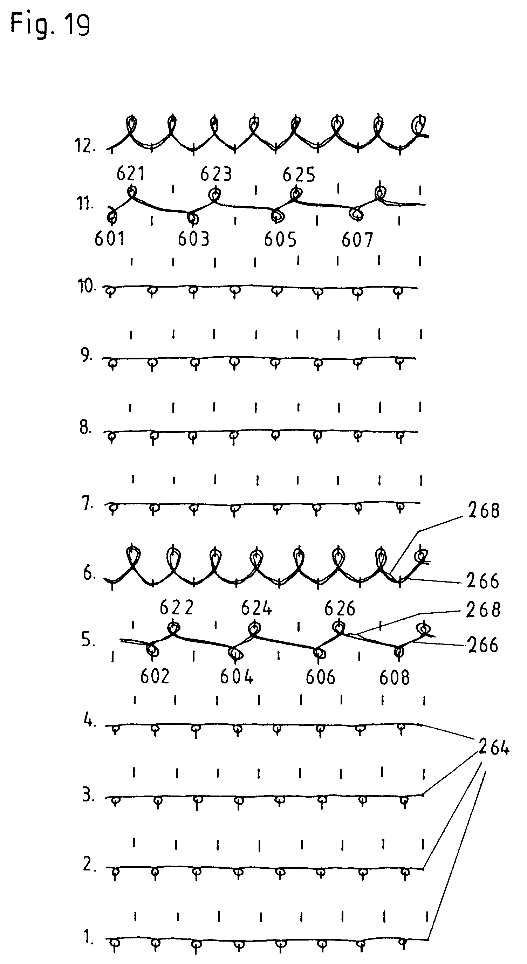

In den Fig. 18 und 19 sind zwei Möglichkeiten aufgezeigt, wie das vorstehend erläuterte Wellengestrick auf automatischen Strickmaschinen hergestellt werden kann. Hierbei verwendet man zwei Nadelbetten, nämlich ein erstes Nadelbett 260 und ein zweites Nadelbett 262 mit im gleichen Abstand zueinander angeordneten Nadeln. Man strickt auf dem ersten Nadelbett 260 mehrere - im gezeigten Ausführungsbeispiel vier - Maschenreihen aus normalem Strickgarn, wie z.B. Baumwoll- oder Polyamidgarn 264. Anschließend werden zwei Maschenreihen auf beiden Nadelbetten 260,262 mit elastischem Garn, wie z.B. Gummi oder Umwindegarn 268 gestrickt, wobei diesem elastischen Garn vorzugsweise ein Plattierfaden 266 beigegeben wird. Bei der fünften Strickreihe wird nur auf ausgewählten Nadeln 602,604,606,.... usw. bzw. 622,624, 626, ... usw. beider Nadelbetten gestrickt. Der Plattierfaden kann von einem Polyamid HE-Faden gebildet sein. Die Darstellung läßt erkennen, daß die Maschenreihe sechs wieder auf allen Nadeln der beiden Nadelbetten 260,262 gestrickt wird. Dies ist jedoch nicht unbedingt erforderlich. Es folgen dann wiederum vier Maschenreihen mit gewöhnlichem Garn auf dem ersten Nadelbett 260 und abschließend zwei weitere Maschenreihen unter Verwendung elastischen Garns, wobei sich die elfte Strickreihe von der fünften dadurch unterscheidet, daß die beteiligten Nadeln der Nadelbetten 260,262 um eins versetzt sind.

Fig. 19 zeigt ein anderes Muster mit einer etwas anderen Bindung im Bereich der späteren Gestrick-Knoten 220. Die Strickreihen eins bis fünf und sieben bis elf entsprechen dem Strickmuster gemäß Fig.18. Unterschiedlich ist lediglich die Ausbildung der Strickreihen 6 bzw. 12, so daß ein näheres Eingehen auf diese Figur nicht erforderlich erscheint.

Abweichend von dem zuvor beschriebenen Herstellungsverfahren ist es auch möglich, zunächst auf einem Nadelbett eine oder mehrere Maschenreihen aus elastischem Garn, wie z.B. Umwindegarn, und auf dem anderen Nadelbett Maschenreihen, aus normalem Strickgarn, wie z.B. Baumwoll- oder Polyamidgarn, zu stricken, wobei anschließend eine oder mehrere Maschenreihen mit allen oder einzelnen Nadeln beider Nadelbetten gestrickt werden.

Das aus textilen Fäden hergestellte Flächengebilde braucht nicht als Gestrick ausgebildet zu sein. Entscheidend ist lediglich, daß das textile Flächengebilde einen solchen Aufbau erhält, daß eine entweder eingearbeitete oder nach vorbestimmtem Muster mit einer textilen Deckstruktur verbundene elastische Fadenanordnung dem Flächengebilde eine solche innere Vorspannung gibt, daß zumindest auf einer Seite ein wellenartiges Relief entsteht, das dann durch äußere Beanspruchung zunächst glatt bzw. glatter gezogen werden kann, ohne bereits in dieser Phase die Deckstruktur auf Dehnung zu beanspruchen.

In Bezug auf ihre Abmessungen sind die beiden Druckpelotten 20,20' unterschiedlich ausgebildet, was auf die auf medialer und lateraler Seite unterschiedlich ausgebildeten und angeordneten Knochenvorsprünge zurückzuführen ist. Die auf medialer Seite liegende Druckpelotte 20 ist dem medialen Knochenvorsprung und die auf lateraler Seite liegende Druckpelotte 20' dem lateralen Knochenvorsprung angepaßt (Fig.1,2,3,7 und 8). Bei einer Länge der Druckpelotte 20 in Originalgröße auf der medialen Seite von etwa 14,5 cm beträgt deren Breite im Bereich der Linie X-X etwa 7 cm (Fig.7), wohingegen bei einer Länge der Druckpelotte 20' auf der lateralen Seite deren Länge im Bereich der Linie X1-X1 etwa 6 cm ist (Fig.8). Auf lateraler Seite ist die Druckpelotte 20' somit schmäler gegenüber der medialen Druckpelotte 20 ausgebildet.

Nach einer weiteren Ausführungsform der Erfindung sind die Druckpelotten 20,20' ohne Friktionskerne 30 an den Seitenteilen 11a,11b des Schlauchkörpers 11 angeordnet. Eine derartige Sprunggelenk-Bandage besteht aus einem anatomisch geformten Schlauchkörper 11 aus einem Gewebe oder Gestrick, der mit einem eine Laufsohle 16a bildenden unteren Abschnitt 16 versehen ist und der im angelegten Zustand den Fuß abschnittsweise umfaßt und bis zur Wade reicht und der zwei anatomisch asymmetrisch ausgebildete, über den paraachillären Weichteilen liegende und am medialen und lateralen Malleolus den Bereich über der Bisgaard'schen Kulisse abdeckende Druckpelotten 20,20' aufweist, von denen jede Druckpelotte 20;20' als Formkörper 21 mit einer seitlichen, abgerundeten Aussparung 27 zur Aufnahme des Knöchelvorsprunges ausgebildet ist, der aus einem weichen oder weich-elastischen oder aus einem harten, inkompressiblen Material besteht, wobei der Formkörper 21 eine flache Grundfläche 22 und eine dem Gelenk zugekehrte, der Grundfläche abgewandte Außenwölbung 23 aufweist, die sich von der einen Längskante 24 zur anderen Längskante 25 unter Ausbildung einer der Längskante 24 benachbarten, wulstartigen Verstärkung konisch verjüngt.

Die hiernach speziell ausgebildeten Druckpelotten 20,20' eignen sich auch ohne Friktionskerne zur Behandlung der Achillodynie, da die Oberflächenprofilierung der Druckpelotten 20,20' voll zur Wirkung kommen.

**Patentansprüche**

1. Sprunggelenk-Bandage aus elastischem Bandagenstoff in Schlauchform, dadurch gekennzeichnet, daß die Bandage (10) aus einem anatomisch geformten Schlauchkörper (11) aus einem Gewebe oder Gestrick besteht, der mit einem eine Laufsohle (16a) bildenden unteren Abschnitt (16) versehen ist und der im angelegten Zustand den Fuß abschnittsweise umfaßt und bis zur Wade reicht und der zwei anatomisch asymmetrisch ausgebildete,über den paraachillären Weichteilen liegende und am medialen und lateralen Malleolus den Bereich über der Bisgaard'schen Kulisse abdekkende Druckpelotten (20,20') aufweist, von denen jede Druckpelotte (20;20') als Formkörper (21) mit einer seitlichen, abgerundeten Aussparung (27) zur Aufnahme des Knöchelvorsprunges ausgebildet ist, der aus einem weichen oder weich-elastischem Material besteht, wobei in dem Formkörper (21) ein stabförmiger Friktionskern (30) aus einem harten, inkompressiblen Material angeordnet und in dem Material des Formkörpers (21) in seiner Lage fixiert ist.

2. Sprunggelenk-Bandage aus elastischem Bandagenstoff in Schlauchform, dadurch gekennzeichnet, daß die Bandage (10) aus einem anatomisch geformten Schlauchkörper (11) aus einem Gewebe oder Gestrick besteht, der mit einem eine Laufsohle (16a) bildenden unteren Abschnitt (16) versehen ist und der im angelegten Zustand den Fuß abschnittsweise umfaßt und bis zur Wade reicht und der zwei anatomisch asymmetrisch ausgebildete, über den paraachillären Weichteilen liegende und am medialen und lateralen Malleolus den Bereich über der Bisgaard'schen Kulisse abdekkende Druckpelotten (20,20') aufweist, von denen jede Druckpelotte (20;20') als Formkörper (21) mit einer seitlichen, abgerundeten Aussparung (27) zur Aufnahme des Knöchelvorsprunges ausgebildet ist, der aus einem weichen oder weich-elastischen oder aus einem harten, inkompressiblen Material besteht, wobei der Formkörper (21) eine flache Grundfläche (22) und eine dem Gelenk zugekehrte, der Grundfläche abgewandte Außenwölbung (23) aufweist, die sich von der einen Längskante (24) zur anderen Längskante (25) unter Ausbildung einer der Längskante (24) benachbarten wulstartigen Verstärkung konisch verjüngt.

3. Sprunggelenk-Bandage nach Anspruch 1 oder 2, dadurch gekennzeichnet, daß zwischen dem die Laufsohle bildenden unteren Abschnitt (16) des Schlauchkörpers (11) und dem oberen Schlauchkörperabschnitt (17) ein zwickelförmiger Einsatz (50) aus einem hochelastischen Relief-Gestrick (51) vorgesehen ist.

4. Sprunggelenk-Bandage nach einem der Ansprüche 1 bis 3, dadurch gekennzeichnet, daß der Schlauchkörper (11) im angelegten Zustand den halben Fuß umfaßt, wobei in Verbindung mit dem Einsatz (50) im Bereich der Laufsohle (16a) eine halbe Ferse (18) eingestrickt ist.

5. Sprunggelenk-Bandage nach einem der Ansprüche 1 und 4, dadurch gekennzeichnet, daß der Schlauchkörper (11) eine etwa L-förmige Formgebung mit einem geringfügig um jeweils anderen Schenkel (15a) abgewinkelten Schenkel (15) aufweist.

6. Sprunggelenk-Bandage nach einem der Ansprüche 1 bis 5, dadurch gekennzeichnet, daß die beiden Druckpelotten (20;20') an den beiden sich gegenüberliegenden Seitenteilen (11a,11b) des Schlauchkörpers (11) angeordnet sind.

7. Sprunggelenk-Bandage nach einem der Ansprüche 1 bis 6, dadurch gekennzeichnet, daß der Formkörper (21) der Druckpelotte (20;20') etwa L-förmig mit einem kurzen, halbkreisförmig auslaufenden Abschnitt (26') und mit einem unter Ausbildung einer abgerundeten, im Bereich der inneren Längskante (25) liegenden Aussparung (27) längeren, in einem etwa keilförmigen spitzen Bereich (28) auslaufenden Abschnitt (29) ausgebildet ist, in seinem vorderen Bereich (25') flach ausläuft, an seinem hinteren Bereich (24') steil abfällt und eine flache Grundfläche (22) aufweist, wobei die Anordnung der beiden Druckpelotten (20,20')

an den Seitenteilen (11a, 11b) des Schlauchkörpers (11) derart ist, daß die Formkörper (21) mit ihren flachen Grundflächen (22) außenliegend sind.

8. Sprunggelenk-Bandage nach einem der Ansprüche 1,3 bis 7, dadurch gekennzeichnet, daß der Friktionskern (30) einer jeden Druckpelotte (20;20') stab- und/oder bogenförmig ausgebildet und benachbart zur äußeren Längskante (24) des Formkörpers (21) im Übergangsbereich des langen Formkörperabschnittes (29) zum kürzeren Formkörperabschnitt (26) angeordnet ist.

9. Sprunggelenk-Bandage nach einem der Ansprüche 1,3 bis 8, dadurch gekennzeichnet, daß der Friktionskern (30) eine gegenüber der Härte des Materials des Formkörpers (21) größere Härte aufweist.

10. Sprunggelenk-Bandage nach einem der Ansprüche 1,3 bis 9, dadurch gekennzeichnet, daß die Differenz zwischen der Härte des Formkörpers (21) und der Härte des Friktionskerns (30) mindestens 10 Shore A, vorzugsweise 20 Shore A, beträgt.

11. Sprunggelenk-Bandage nach einem der Ansprüche 1,3 bis 10, dadurch gekennzeichnet, daß das Material des Formkörpers (21) eine unterhalb 50 Shore A liegende Härte und das Material des Friktionskerns (30) eine oberhalb 50 Shore A liegende Härte aufweist.

12. Sprunggelenk-Bandage nach einem der Ansprüche 1 3 bis 11, dadurch gekennzeichnet, daß der Friktionskern (30) zur Lagenfixierung in dem Formkörper (21) der Druckpelotte (20;20') im Bereich seiner umlaufenden Wandfläche (31) eine rillenförmige Ausnehmung, Einziehung, wie Nut, Hinterschneidungen, Verzahnung od.dgl., (32) zur Aufnahme von Formkörpermaterial aufweist.

13. Sprunggelenk-Bandage nach einem der Ansprüche 1,3 bis 12, dadurch gekennzeichnet, daß das Material des Friktionskerns (30) mit dem Material des Formkörpers (21) verschmolzen und mit dem Formkörpermaterial unlösbar verbunden ist, wobei der Friktionskern (30) und der Formkörper (21) aus Kunststoffen, insbesondere Silikonkautschuken od.dgl, besteht.

14. Sprunggelenk-Bandage nach einem der Ansprüche 1,3 bis 12, dadurch gekennzeichnet, daß der Friktionskern (30) bei der Herstellung des Formkörpers (21) durch Materialaushärtung eines Abschnittes des Formkörpers (21) erhalten wird, wobei der Friktionskern (30) und der Formkörper (21) aus Kunststoffen, insbesondere Silikonkautschuken od.dgl., unterschiedlicher Härtegrade besteht.

15. Sprunggelenk-Bandage nach einem der Ansprüche 1,3 bis 14, dadurch gekennzeichnet, daß das Formkörper (21) eine flache Grundfläche (22) und eine dem Gelenk zugekehrte, der Grundfläche abgewandte Außenwölbung (23) aufweist.

16. Sprunggelenk-Bandage nach Anspruch 15, dadurch gekennzeichnet, daß die Außenwölbung (23) des Formkörpers (21) sich von der einen Längskante (24) zur anderen Längskante (25) konisch verjüngt und daß der Friktionskern (30) in dem Bereich der Außenwölbung (23) angeordnet ist, der den größten Querschnitt aufweist.

17. Sprunggelenk-Bandage nach einem der Ansprüche 1 bis 16, dadurch gekennzeichnet, daß der Formkörper (21) aus Filz, Moosgummi, Neopren, Gummi, einem viskoelastischen Silikonkautschuk oder einem elastischen, kompressiblen, durch Druck verformbaren Silikonkautschuk oder einem Material mit gleichen Elastizitätseigenschaften, wie Naturkautschuk, Silikonschaum od.dgl., besteht.

18. Sprunggelenk-Bandage nach einem der Ansprüche 1 bis 17, dadurch gekennzeichnet, daß der Formkörper (21) beutelförmig ausgebildet ist und eine Füllung aus einem gasförmigen oder flüssigen Medium aufweist.

19. Sprunggelenk-Bandage nach einem der Ansprüche 1,3 bis 18, dadurch gekennzeichnet, daß bei einer beutelförmigen Ausbildung des Formkörpers (21) der Friktionskern (30) an der Innenwandfläche des Beutels in seiner Lage fixiert ist.

20. Sprunggelenk-Bandage nach einem der Ansprüche 1,3 bis 19, dadurch gekennzeichnet, daß der Friktionskern (30) aus einem inkompressiblen Kunststoff, wie einem natürlichen oder künstlichen Kautschuk, Hartgummi,Chloropren-Polymerisat, einem gummielastischen, vernetzten Polyurethan,Polyamid, Metall, Holz od.dgl. besteht.

21. Sprunggelenk-Bandage nach einem der Ansprüche 1,3 bis 20, dadurch gekennzeichnet, daß in dem Formkörper (21) der Druckpelotte (20;20') eine den Friktionskern (30) aufnehmende Ausnehmung (40) ausgebildet ist.

22. Sprunggelenk-Bandage nach Anspruch 21, dadurch gekennzeichnet, daß der Friktionskern (30) in der Ausnehmung (40) lösbar mittels Preß- oder Klemmsitz gehalten ist.

23. Sprunggelenk-Bandage nach einem der Ansprüche 1,3 bis 22, dadurch gekennzeichnet, daß die obere Wandfläche des Friktionskerns (30) bogenförmig, halbkreisförmig oder flach mit abgerundeten Ecken ausgebildet ist.

24. Sprunggelenk-Bandage nach einem der Ansprüche 1 bis 23, dadurch gekennzeichnet, daß der Schlauchkörper (11) im einseitig aufgetrennten und entfalteten Zustand aus zwei deckungsgleichen, im Bereich einer Längsseite miteinander verbundenen Seitenteilen (11a,11b) besteht, wobei zur Bildung der Schlauchform die beiden Seitenteile (11a,11b) im Bereich ihrer freien Längskanten über eine Längsnaht (12) miteinander verbunden sind.

25. Sprunggelenk-Bandage nach einem der Ansprüche 1 bis 24, dadurch gekennzeichnet, daß die auf medialer Seite in dem Seitenteil (11a) des Schlauchkörpers (11) angeordnete Druckpelotte (20) gegenüber der auf lateraler Seite in dem Seitenteil (11b) angeordnete Druckpelotte (20') eine größere Breite aufweist.

26. Sprunggelenk-Bandage nach einem der Ansprüche 3 bis 25, dadurch gekennzeichnet, daß der Einsatz (50) in dem Schlauchkörper (11) aus einem aus textilen Fäden hergestellten Flächengebilde mit einer zumindest auf einer Seite ausgebildeten Querwellenstruktur (218) besteht, die durch eine eingearbeitete oder unter einer Deckstruktur liegende elastische Fadenanordnung (226) elastisch vorgespannt und stabilisiert ist, die in vorbestimmten Abständen (A) mit der Deckstruktur verbunden ist.

27. Sprunggelenk-Bandage nach einem der Ansprüche 3 bis 26 dadurch gekennzeichnet, daß die Deckstruktur des Flächengebildes von einem einflächigen Deckgestrick (224) gebildet ist und die Querwellen (218) jeweils mehrere Maschen (222) aufweisen.

28. Sprunggelenk-Bandage nach Anspruch 27, dadurch gekennzeichnet, daß das Deckgestrick (224) aus einer im wesentlichen unelastischen Maschenware (264) besteht.

29. Sprunggelenk-Bandage nach einem der Ansprüche 3 bis 28, dadurch gekennzeichnet, daß die elastische Fadenanordnung (226) zusammen mit dem Deckgestrick (224) in einem Arbeitsgang hergestellt wird.

30. Sprunggelenk-Bandage nach einem der Ansprüche 3 bis 29, dadurch gekennzeichnet, daß die Querwellenstruktur aus Halbwellen besteht, die jeweils zwischen zwei und zwölf, vorzugsweise vier, Maschenreihen aufweisen.

31. Sprunggelenk-Bandage nach einem der Ansprüche 3 bis 30, dadurch gekennzeichnet, daß das Deckgestrick (224) aus Baumwoll- und/oder Polyamid-Garn (264) besteht.

32. Sprunggelenk-Bandage nach einem der Ansprüche 3 bis 31, dadurch gekennzeichnet, daß die elastische Fadenanordnung (226) aus Umwindegarn (268) besteht oder dieses aufweist.

33. Sprunggelenk-Bandage nach Anspruch 32, dadurch gekennzeichnet, daß das Umwindegarn (268) plattiert ist.

34. Sprunggelenk-Bandage nach einem der Ansprüche 3 bis 33, dadurch gekennzeichnet, daß in das Deckgestrick (224) ein Einlegefaden eingearbeitet ist.

35. Sprunggelenk-Bandage nach einem der Ansprüche 3 bis 34, dadurch gekennzeichnet, daß die Querwellenstruktur (218) im wesentlichen senkrecht zur Haupt-Dehnungsrichtung (F) ausgerichtet ist.

36. Sprunggelenk-Bandage nach Anspruch 35, dadurch gekennzeichnet, daß das Gestrick (51) derart geformt ist, daß die Anzahl der Querwellen (218) dort am größten ist, wo aufgrund der Gelenkbeugung der größte Dehnungsweg (L) auftritt.

# Fig.1

Fig. 2

Fig. 4

Fig. 5

Fig. 6

Fig. 8

Fig.7

Fig. 9

Fig. 10

Fig. 11

Fig. 12

FIG. 13

Fig. 14

Fig. 15

FIG.16

Fig. 3

Fig.17

# Fig.18

EP 0 499 710 A2

## Fig. 19